# EUROPEAN PATENT SPECIFICATION

(11) **EP 3 729 088 B1**
(45) Date of publication and mention of the grant of the patent: **20.10.2021**
(21) Application number: 18833205.0
(22) Date of filing: 19.12.2018
(51) Int. Cl.: G01N 33/542, G01N 33/58, G01N 33/68

(54) **DIAGNOSIS AND TREATMENT OF ALPHA-SYNUCLEINOPATHIES**
DIAGNOSE UND ÜBERWACHUNG VON ALPHA-SYNUKLEINOPATHIEN
DIAGNOSTIC ET SUIVI DE LA MALADIE D'ALPHA-SYNUCLÉINOPATHIES

(30) Priority: 21.12.2017 DK PA201700738
(43) Date of publication of application: 28.10.2020
(73) Proprietor: H. Lundbeck A/S, 2500 Valby (DK)
(72) Inventor: MALIK, Ibrahim, John, 2500 Valby (DK); KALLUNKI, Pekka, 2500 Valby (DK); FOG, Karina, 2500 Valby (DK)
(86) International application number: PCT/EP2018/085898
(87) International publication number: WO 2019/121952

(56) References cited:
- US-A1- 2014 186 294
- US-A1- 2017 192 017
- ANJA HVIID SIMONSEN ET AL: "The utility of [alpha]-synuclein as biofluid marker in neurodegenerative diseases: a systematic review of the literature", BIOMARKERS IN MEDICINE, vol. 10, no. 1, 1 January 2016 (2016-01-01), pages 19-34, XP055564944, UK ISSN: 1752-0363, DOI: 10.2217/BMM.14.105
- HONGLI ZHAO ET AL: "AlphaLISA detection of alpha-synuclein in the cerebrospinal fluid and its potential application in Parkinson's disease diagnosis", PROTEIN & CELL, vol. 8, no. 9, 29 May 2017 (2017-05-29), pages 696-700, XP055564947, Beijing, CN ISSN: 1674-800X, DOI: 10.1007/s13238-017-0424-4
- BENJAMIN DEHAY ET AL: "Targeting [alpha]-synuclein for treatment of Parkinson's disease: mechanistic and therapeutic considerations", LANCET NEUROLOGY, vol. 14, no. 8, 1 August 2015 (2015-08-01) , pages 855-866, XP055564986, GB ISSN: 1474-4422, DOI: 10.1016/S1474-4422(15)00006-X
- Fran?ois Degorce ET AL: "HTRF: A Technology Tailored for Drug Discovery -A Review of Theoreti- cal Aspects and Recent Applications", Current Chemical Genomics, 1 January 2009 (2009-01-01), pages 22-32, XP055129390, DOI: 10.2174/1875397300903010022 Retrieved from the Internet: URL:http://www.ncbi.nlm.nih.gov/pmc/articl es/PMC2802762/pdf/TOCHGENJ-3-22.pdf [retrieved on 2014-07-16]

### SEQUENCE LISTING

<110> H. Lundbeck A/S
<120> Assay, method and treatment of alpha-synucleinopathies
<130> 1098-WO-PCT
<160> 1
<170> PatentIn version 3.5
<210> 1
   <211> 140
   <212> PRT
   <213> Homo sapiens
<400> 1

## Description

### Field of the invention

The present invention relates to an Time Resolved Fluorescence Resonance Energy Transfer (TR-FRET) assay that enables diagnosis and monitoring of treatment response of a medicament used to treat alpha-synucleinopathies such as Parkinson's Disease patients by detecting alpha-synuclein levels in patients.

### Background of the invention

Parkinson's disease (PD), Parkinson's disease with dementia (PDD), dementia with Lewy bodies (DLB) and multiple systems atrophy (MSA) are examples of neurodegenerative disorders with α-synuclein brain pathology. PD is the most common movement disorder and is characterized by tremor, bradykinesia or difficulty to initiate movements, rigidity and impairment of balance also referred to as postural instability. PD is believed to affect approximately four to six million people worldwide. About 80% of PD patients develop dementia leading to PDD. Typically, dementia occurs late in the course of Parkinson's disease. In DLB, dementia is the first symptom, while motor symptoms may appear in the first year, making this the clinical distinction between these disorders. DLB may represent up to 15-20% of all dementia.

Alpha-synuclein is a small 140 amino acid intraneuronal protein, predominantly located presynaptically. Intraneuronal accumulation of α-synuclein results in the formation aggregates inside neurons, such as Lewy bodies and pale bodies, which are large round cytoplasmic inclusions, Lewy neurites, which are thread like inclusions in axons, and small synaptic inclusions.

The process leading to α-synuclein aggregation and toxicity is not well understood. Mutations or duplications of the α-synuclein gene are rare cause of PD and DLB. The pathogenic mutations A30P, A53T, E46K (Kruger et al., 1998) (Polymeropoulos et al., 1998) (Zarranz et al., 2004) and duplication and triplication of the α-synuclein (Chartier-Harlin et al. 2004)(Singleton et al., 2003) have been reported to cause either PD or DLB. Most of these mutations have been linked to increase in the rate of protofibrillar and finally fibrillar species are formed, and these may have different toxic properties. Aggregation is associated with phosphorylation of alpha-synuclein at serine 129. While normal alpha-synuclein is only phosphorylated to small extent (4%) the fibrillary alpha-synuclein is estimated to be 80% phosphorylated, and antibodies to pS129 alpha-synuclein detect the smallest aggregates.

Small amounts of alpha-synuclein are found extracellularly in interstitial fluid in brain, in Cerebrospinal fluid (CSF) and blood. The source of this alpha-synuclein is not clear, but it is likely released from cells, both from cell bodies and synapses, for example during synaptic release when neuronal cells are activated. Some of it is also likely coming from diseased and dead cells. Alpha-synuclein is also found in many blood cells and platelets, which are a likely source of alpha-synuclein in blood. Small proportion of alpha-synuclein is secreted in exosomes, while majority of alpha-synuclein in extracellular fluid is found as free protein not associated with exosomes.

There is a need for improved diagnostic tools to identify patients at early stages of a neurodegenerative disease with α-synuclein pathology. There is evidence that the neurodegenerative process starts years, perhaps 10-20 years before clinical diagnosis. REM sleep behaviour disorder (RBD) is now recognized as the prodromal stage of an α-synucleinopathy. Most people with RDB will convert to PD or DLB within the next 20 years from diagnosis of RBD. Other signs of prodromal stage of alpha-synucleinopathy include loss of olfaction.

Today there are no biochemical methods to aid a clinician's diagnosis before the motor symptoms are evident. At that point, substantial damage to the brain has probably already occurred. The importance of accurate diagnostic assays will become even greater as new disease modifying therapies emerge, that will hopefully stop or slow the progression of the disease.

Several assays measuring the total alpha-synuclein are available, and some of them are validated or are being validated in large clinical studies (Goldman et al. 2017). These studies have shown a small decrease in total alpha-synuclein in CSF to be associated with Parkinson's disease. Several exploratory assays to measure some kind of oligomeric forms of alpha-synuclein, and phosphorylated forms on Serine-129 also exist (Majbour et al. 2016a), and preliminary data suggests that these species may be increased in PD CSF. However, again the difference between patient and control levels are small and there is a large interindividual variation. All of the current assays suffer from the small difference and large interindividual variation, making these assays not useful in the help for diagnosis for diseases with alpha-synuclein aggregation. All in all, the current evidence suggests that total CSF α-synuclein is reduced in PD and that subspecies of α-synuclein oligomers, or phosphorylated alpha-synuclein may distinguish PD from controls.

Measuring alpha-synuclein in peripheral biofluids, blood plasma and saliva has produced even more variable results than CSF. In latest large study plasma and saliva α-synuclein levels did not significantly differentiate PD from healthy control participants and there was no significant correlation of α-synuclein levels in peripheral biofluids (plasma and saliva) with α-synuclein levels in CSF (Goldman et al. 2017). Therefore, currently CSF α-synuclein is of greater diagnostic utility for PD than peripheral α-synuclein. CSF sampling is more invasive way for obtaining samples, and it would be beneficial to have plasma biomarker for PD, if this was possible.

Hongli Zhao et al. has described the use of Time Resolved Fluorescence Resonance Energy Transfer (TR-FRET) to detect alpha synuclein in CSF (Zhao et al, 2017).

### Summary of the invention

The present invention relates to an in vitro method for diagnosing alpha-synucleinopathies or for monitoring the treatment response of a medicament used to treat alpha-synucleinopathies by detecting alpha-synuclein in a sample of a subject comprising the steps of:
a) providing a sample obtained from a subject,
b) Applying the sample to a luminescence assay, and
c) Optionally, comparing the results with a control,
wherein in said sample is a blood sample, a plasma sample or a serum sample, and
wherein the luminescence assay uses Time Resolved Fluorescence Resonance Energy Transfer (TR-FRET) in which two different fluorophores are linked to two antibodies binding to alpha-synuclein or pS129 (phospho-serine 129) of alpha-synuclein, wherein one fluorophore used has a longer fluorescence time (donor) than the other fluorophore used (acceptor).

The in vitro method of the invention can be used for detecting aggregated alpha-synuclein in a sample, wherein the luminescence assay is using two identical alpha-synuclein antibodies binding to alpha-synuclein.

The in vitro method of the invention can be used for detecting phosphorylated alpha-synuclein in a sample, wherein the luminescence assay is using two different alpha-synuclein antibodies, wherein one antibody is binding within the 120-140 residues on alpha-synuclein and the other antibody is binding pS129 of alpha-synuclein.

In one embodiment the in vitro method of the invention, the donor fluorophore is selected from Lumi4-Tb (Tb2+ cryptate) or Europium cryptate (Eu3+ cryptate).

In another embodiment, the in vitro method of the invention, the acceptor fluorophore is selected from d2, XL665, or fluorescein.

The in vitro method according to the invention, wherein the subject is a human.

### Drawings

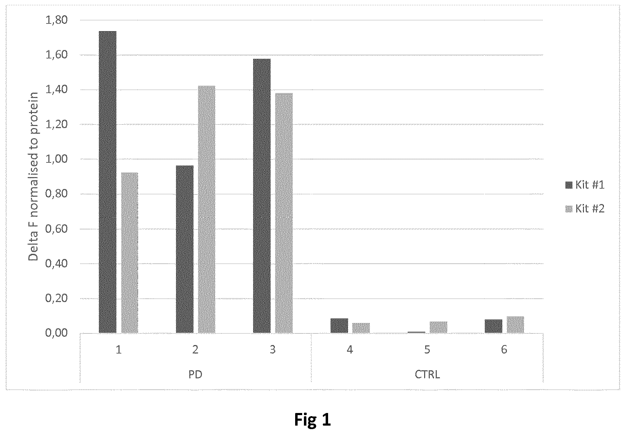

**Figure 1** shows measurements done using PHERAstar FSX device, which allows for simultaneous measurement of both 620nM (Tb-cryptate - donor) and 665nm (d2-acceptor) emissions. Ratio of 665/620^{∗}10000 is calculated for each well. The relative energy transfer rate, Delta F %, is calculated using the ratio and can be normalized to protein concentration. The column graph shows the Delta F % normalized to protein for each of the plasma sample (diluted eight times) measured using both kit #1 and kit # 2 (detecting aggregated alpha synuclein). There is a clear distinction between the three PD patients and the three healthy controls. This figure shows that there is a significantly high level of aggregated alpha synuclein in the plasma of PD patients and this method can be used to identify and diagnose and monitor disease progression in PD patients based on a blood sample.

### Detailed description of the invention

The object of the present invention is to provide a Time Resolved Fluorescence Resonance Energy Transfer (TR-FRET) assay for use in diagnostic methods for alpha-synuclein related disorders, *i*.*e*., alpha-synucleinopathies wherein accumulation of aggregated insoluble alpha-synuclein in the form of Lewy bodies, Lewy neurites and small synaptic inclusions, are present in the brain. Such disorders include, but are not limited to, one or more neurodegenerative disorders such as Parkinson's disease (PD), Parkinson's disease with dementia (PDD), dementia with Lewy bodies (DLB), multiple system atrophy (MSA), and REM sleep behavioural disorder (RBD) and other neurodegenerative disorders with alpha-synuclein pathology.

The present invention provides new use for two Time Resolved Fluorescence Resonance Energy Transfer (TR-FRET) assays, one based on aggregated alpha-synuclein and one on phosphorylated alpha-synuclein. Both assays show large increase in plasma from PD patients compared to plasma from control individuals (FIG 1).

The treatment response of an antibody treatment for alpha-synucleinopathies may be evaluated measuring the patients level of alpha-synuclein in blood, plasma or serum. The evaluation may be made prior to antibody treatment (e.g. when diagnosing the patient) or after 1, 2, 3, 4 or more treatments. For example, the method may according to one embodiment also be used to monitor the treatment response of the antibody treatment and the disease progression.

The assay comprise the steps of
a. Applying a blood, plasma or serum sample from a patient to a Time Resolved Fluorescence Resonance Energy Transfer (TR-FRET) assay under appropriate binding conditions, wherein the Time Resolved Fluorescence Resonance Energy Transfer (TR-FRET) assay uses two different fluorophores linked to two antibodies binding to alpha-synuclein or pS129 (phospho-serine 129) of alpha-synuclein, wherein one fluorophore used has a longer fluorescence time (donor) than the other fluorophore used (acceptor), and
b. if applicable, comparing the data with a control from a person that do not have Parkinson's Disease or for example, comparing the data with data obtained at a different (earlier) timepoint from the same person, and thereby monitor the disease progression

Based on the result it may be determined whether or not the patient shall continue treatment, or if just diagnosed if the patient is to initiate treatment. For example, if the data obtained by the assay shows more alpha-synuclein present compared to the control continued or initiation of treatment may be advised.

The difference between the control and the patient may be more the 2-fold, 3-fold, 4-fold or above. As shown in Figure 1, the control is very low and the thus a positive result may be found by disregarding the background.

The assay is a HTRF (Homogeneous Time Resolved Fluorescence) based assay. This technology combines fluorescence resonance energy transfer technology (FRET) with time-resolved measurement (TR) (Degorce et al, 2009, current Chemical Genomics, 3, 22-32). In the TR-FRET assays, a signal is generated through fluorescent resonance energy transfer between donor and an acceptor molecule (e.g. coupled to an antibody) when in close proximity to each other.

HTRF technology may use Europium cryptate as a fluorescence donor to monitor reactions between biomolecules such as antibodies or Terbium cryptate (Tb). Examples include Europium cryptate (Eu3+ cryptate) and Lumi4-Tb (Tb2+ cryptate).

An acceptor developed for HTRF may be XL665, a phycobiliprotein pigment purified from red algae. XL665 is a large heterohexameric edifice of 105 kDa, cross-linked after isolation for better stability and preservation of its photo physical properties in HTRF assays. A type of acceptor that possesses a series of photo physical properties very similar to those of XL665 but is characterized by organic structures which are 100 times smaller than XL665 are e.g. earth chelate or cryptate. By using smaller entities this solves the steric hindrance problems sometimes suspected in XL665 based TR-FRET systems. These near-infrared acceptors are also particularly suited for homogeneous assays since their emission is less likely to be disturbed by intrinsic medium or compound auto fluorescence arise in the typical compound screen process. The properties of these red acceptors also make them suitable for coupling with Terbium cryptate. Moreover, due to additional peaks in its emission spectrum, Terbium cryptate can be coupled with green acceptors such as fluorescein, emitting in the 520 nm range that may for instance allow designing multiplex assays with two readouts.

In particular, a fluorescent compound such as a rare earth chelate or cryptate will advantageously be used, especially a terbium, europium, dysprosium, samarium or neodymium chelate or cryptate. A terbium or europium cryptate will preferably be used.

In the fluorescent methods of detection and/or determination using the method of measurement of the invention, a rare earth cryptate described in European patent applications EP180 492 and EP321 353 will advantageously be chosen.

The terbium cryptate Tb trisbipyridine or the europium cryptate Eu trisbipyridine, as described in European patent application 180 492, or the cryptares Eu trisbipyridinediamine and Tb trisbipyridinediamine, described in European patent application EP321 353, will preferably be used.

According to an advantageous feature, the fluorescent donor compound is a europium cryptate and the fluorescent acceptor compound is selected from d2, allophycocyanin, allophycocyanin B, phycocyanin C or phycocyanin R.

It is also possible to use a phosphorescent compound, such as eosin or erythrosine, as the luminescent donor. In this case, it will be advantageous to use a fluorescent acceptor compound selected from chlorophylis such as those mentioned in European patent applications EP71 991 and EP314 406, or porphyrins such as those mentioned in European patent application EP71 991, or else phthalocyanins such as those of international patent application WO 88 04777.

According to another feature of the invention, the Time Resolved Fluorescence Resonance Energy Transfer (TR-FRET) assay for detecting and/or determining alpha-synuclein in a blood, plasma or serum sample comprising the steps of:
1) adding, to said sample containing alpha-synuclein from the patient or subject, a first antibody against alpha-synuclein, coupled with a luminescent donor,
2) adding a second alpha-synuclein antibody coupled with a luminescent acceptor,
3) incubating said medium after the addition of reagents
4) exciting the resulting medium at the excitation wavelength of the luminescent donor, and
5) measuring the signal of the luminescent donor at a wavelength (this measurement serving as a reference), and the signal resulting from the energy transfer at a different wavelength.

According to one embodiment, an assay is intended for the detection of alpha-synuclein aggregation using the HTRF technology. Aggregated alpha-synuclein is detected using specific alpha-synuclein monoclonal antibodies, labelled either a donor such as Tb-Cryptate or with an acceptor. When the dyes are in close proximity, the excitation of the donor with a light source (laser or flash lamp) triggers a Fluorescence Resonance Energy Transfer (FRET) towards the acceptor, which in turn fluoresces at a specific wavelength (665nm). The antibody labelled with acceptor or Tb binds to alpha-synuclein. When alpha-synuclein aggregates, the antibody labelled with acceptor or Tb come then into a close proximity generating FRET. Signal intensity is proportional to the number of aggregates formed.

Monitoring alpha-synuclein aggregation is of great interest for studying the pathogenesis of synucleinopathies, a group of neurodegenerative diseases that includes Parkinson's disease (PD), dementia with Lewy bodies (DLB), diffuse Lewy body disease (DLBD), and multiple system atrophy (MSA). Accordingly, the method of the present invention can be used to diagnose or monitor the diseases mentioned above. Alternatively, the method can be used to monitor or follow the treatment response and to take decision related to treatment of the patients. In one embodiment, this treatment is alpha-synuclein antibody treatment.

Thus, the present invention relates to method of diagnose or follow synucleinopathies such as Parkinson Disease in patients comprising the steps of
a) Obtaining a sample from a patient
b) Applying the sample on a luminescence assay, as described above, and
c) Optionally, comparing the results with a control,
wherein said sample is a blood sample, a plasma sample or a serum sample, and
wherein the luminescence assay uses Time Resolved Fluorescence Resonance Energy Transfer (TR-FRET) in which two different fluorophores are linked to two antibodies binding to alpha-synuclein or pS129 (phospho-serine 129) of alpha-synuclein, wherein one fluorophore used has a longer fluorescence time (donor) than the other fluorophore used (acceptor).

The antibodies used are alpha-synuclein antibodies linked a fluorophore. The fluorophore used has a longer fluorescence time (donor) than the other fluorophore used (acceptor).

The donor is preferably selected from Lumi4-Tb (Tb2+ cryptate) or Europium cryptate (Eu3+ cryptate), and the acceptor is preferably selected from d2, XL665, or fluorescein.

The proximity between the donor and acceptor is assessed by detecting the level of energy transfer by measuring the fluorescence emission, preferably at two different wavelengths (such as 665 nm and 620 nm) in a compatible reader

As used herein, the term "alpha-synuclein" is synonymous with "the alpha-synuclein protein" and refers to any of the alpha-synuclein protein isoforms (identified in, for example, UniProt as P37840, 1-3). The amino acid numbering of alpha-synuclein is given with respect to the sequence as shown below, with methionine (M) being amino acid residue1 (SEQ ID NO: 1):

The alpha synuclein antibodies may in some embodiments be binding the C-terminal residues between 120-140 of alpha synuclein or pS129 alpha-synuclein.

### Example

This assay can be used to identify, diagnose and monitor patients suffering from alpha synucleinopathy, e.g. Parkinson Disease, using a blood sample.

### Assay principle:

This assay is based on using a Time resolved ― Fluorescence Resonance Energy Transfer, (TR-FRET) technology.

FRET is based on transfer of energy between two dyes, a donor and an acceptor. When the donor is excited, it transfers energy to the acceptor which in turn emits fluorescence that is measured. This is only possible if the dyes are very close to each other. Normal FRET studies are limited by background fluorescence which are extremely transient. This can be overcome by combining Time resolved measurements with FRET to avoid background and non-specific fluorescence. Also, the acceptors in TR-FRET are designed to emit long-lived fluorescence when they are involved in FRET.

In the alpha synuclein aggregation kit, a specific monoclonal antibody is labelled with either a donor or an acceptor molecule. In the Phospho-synuclein kit (S129P), two antibodies are used, one is an alpha synuclein antibody and the other a Phospho-synuclein specific antibody (S129P), where one of the antibodies is labelled with a donor and the other with an acceptor dye. When the antibodies (labelled with donor and acceptor dyes) bind to human alpha synuclein aggregates, they come very close to each other and generate FRET upon excitation.

### Patients

Patients were recruited from the outpatient clinic of the Department of Neurology, Bispebjerg-Frederiksberg Hospitals. The clinical diagnosis for PD was defined according to UK Parkinson's Disease Society Brain Bank clinical diagnostic criteria. Patients were included consecutively and a clinical follow-up was performed for all patients. At follow-up, only those patients fulfilling the diagnostic criteria for a definite PD diagnosis were accepted in the study. Subjects from the control group were free of diseases that might affect the central nervous system.

### Plasma samples

Venous blood was drawn at the respective clinics and processed on the same day at Bispebjerg Movement Disorders Biobank, Copenhagen, DK. All plasma samples were collected at inclusion. Samples were collected in EDTA coated polypropylene tubes, and were spun at 2000 x g for 10 minutes at 4°C; the supernatant plasma was then aliquoted and stored in 400 µL polypropylene (PP) tubes at -80 °C until the day of analysis, when they were thawed on ice for 30 min.

### Example 1

Samples from patients (3x controls and 3x PD pts) were analyzed. Two commercial kits from Cisbio were used, alpha synuclein aggregation kit (Kit #1) and a Phospho-synuclein kit (Kit #2), to compare the samples.). Each sample was tested in three different dilutions. The samples were diluted in the buffer provided with the kits and pipetted into a 386 well plate in duplicates. The antibody mixture is added to the well and incubated at RT. Positive and negative controls are included in the plate.

Measurements were done using PHERAstar FSX device, which allows for simultaneous measurement of both 620nM (Tb-cryptate - donor) and 665nm (d2- acceptor) emissions. Ratio of 665/620^{∗}10000 is calculated for each well. The relative energy transfer rate, Delta F %, is calculated using the ratio and can be normalized to protein concentration. The column graph shows the Delta F % normalized to protein for each of the sample (diluted eight times) measured using both kit #1 and kit #2. There is a clear distinction between the three PD patients and the three healthy controls. This example shows that there is a significantly high level of aggregated alpha synuclein and phospho-synuclein in the plasma of PD patients and this method can be used to identify and diagnose PD cases based on a blood sample.

## Claims

1. An in vitro method for diagnosing alpha-synucleinopathies or for monitoring the treatment response of a medicament used to treat alpha-synucleinopathies by detecting alpha-synuclein in a sample of a subject comprising the steps of:
a) providing a sample obtained from a subject,
b) Applying the sample to a luminescence assay, and
c) Optionally, comparing the results with a control,
wherein said sample is a blood sample, a plasma sample or a serum sample, and wherein the luminescence assay uses Time Resolved Fluorescence Resonance Energy Transfer (TR-FRET) in which two different fluorophores are linked to two antibodies binding to alpha-synuclein or pS129 (phospho-serine 129) of alpha-synuclein (the numbering of amino acids being in accordance with SEQ ID NO: 1), wherein one fluorophore used has a longer fluorescence time (donor) than the other fluorophore used (acceptor).

2. The in vitro method according to claim 1 for detecting aggregated alpha-synuclein in a sample, wherein the luminescence assay is using two identical alpha-synuclein antibodies binding to alpha-synuclein.

3. The in vitro method according to claim 1 for detecting phosphorylated alpha-synuclein in a sample, wherein the luminescence assay is using two different alpha-synuclein antibodies, wherein one antibody is binding within the 120-140 residues on alpha-synuclein and the other antibody is binding pS129 of alpha-synuclein.

4. The in vitro method according to any of claims 1-3, wherein the donor fluorophore is selected from Lumi4-Tb (Tb2+ cryptate) or Europium cryptate (Eu3+ cryptate).

5. The in vitro method according to any of claims 1-4, wherein the acceptor fluorophore is selected from d2, XL665, or fluorescein.

6. The method according to any one of the preceding claims, wherein the subject is a human.

## Patentansprüche

1. In-vitro-Verfahren zum Diagnostizieren von alpha-Synucleinopathien oder zum Überwachen der Behandlungsreaktion eines Medikaments, das verwendet wird, um alpha-Synucleinopathien zu behandeln, durch Nachweisen von alpha-Synuclein in einer Probe eines Probanden, umfassend die Schritte des:
a) Bereitstellens einer von einem Probanden erhaltenen Probe,
b) Anwendens der Probe an einem Lumineszenzassay und
c) wahlweise, Vergleichens der Ergebnisse mit einer Kontrolle,
wobei die Probe eine Blutprobe, eine Plasmaprobe oder eine Serumprobe ist, und
wobei der Lumineszenzassay zeitaufgelösten Fluoreszenzresonanz-Energietransfer (TR-FRET) verwendet, in dem zwei unterschiedliche Fluorophore mit zwei Antikörpern verknüpft sind, die an alpha-Synuclein oder pS129 (Phosphoserin 129) von alpha-Synuclein binden (wobei die Nummerierung von Aminosäuren in Übereinstimmung mit SEQ ID NO: 1 ist),
wobei
ein verwendeter Fluorophor eine längere Fluoreszenzzeit (Donor) aufweist als der andere verwendete Fluorophor (Akzeptor).

2. In-vitro-Verfahren nach Anspruch 1 zum Nachweisen aggregierten alpha-Synucleins in einer Probe, wobei der Lumineszenzassay zwei identische alpha-Synuclein-Antikörper, die an alpha-Synuclein binden, verwendet.

3. In-vitro-Verfahren nach Anspruch 1 zum Nachweisen phosphorylierten alpha-Synucleins in einer Probe, wobei der Lumineszenzassay zwei unterschiedliche alpha-Synuclein-Antikörper verwendet, wobei ein Antikörper innerhalb der 120-140-Reste an alpha-Synuclein bindet und der andere Antikörper pS129 von alpha-Synuclein bindet.

4. In-vitro-Verfahren nach einem der Ansprüche 1-3, wobei der Donorfluorophor ausgewählt ist aus Lumi4-Tb (Tb2+-Kryptat) oder Europiumkryptat (Eu3+-Kryptat).

5. In-vitro-Verfahren nach einem der Ansprüche 1-4, wobei der Akzeptorfluorophor ausgewählt ist aus d2, XL665 oder Fluorescein.

6. Verfahren nach einem der vorstehenden Ansprüche, wobei der Proband ein Mensch ist.

## Revendications

1. Procédé in vitro de diagnostic d'alpha-synucléinopathies ou de surveillance de la réponse à un traitement avec un médicament utilisé pour traiter les alpha-synucléinopathies par la détection d'alpha-synucléine dans un échantillon d'un sujet comprenant les étapes consistant à :
a) fournir un échantillon obtenu à partir d'un sujet,
b) appliquer l'échantillon à un dosage par luminescence, et
c) facultativement, comparer les résultats à un témoin,
dans lequel ledit échantillon est un échantillon de sang, un échantillon de plasma ou un échantillon de sérum, et dans lequel le dosage par luminescence utilise un transfert d'énergie de fluorescence par résonance à résolution temporelle (TR-FRET) dans lequel deux fluorophores différents sont liés à deux anticorps se liant à l'alpha-synucléine ou à pS129 (phospho-sérine 129) de l'alpha-synucléine (la numérotation des acides aminés étant conforme à SEQ ID NO : 1), dans lequel un fluorophore utilisé présente une durée de fluorescence plus longue (donneur) que l'autre fluorophore utilisé (accepteur).

2. Procédé in vitro selon la revendication 1 pour détecter une alpha-synucléine agrégée dans un échantillon, dans lequel le dosage par luminescence utilise deux anticorps anti-alpha-synucléine identiques se liant à l'alpha-synucléine.

3. Procédé in vitro selon la revendication 1 pour détecter une alpha-synucléine phosphorylée dans un échantillon, dans lequel le dosage par luminescence utilise deux anticorps anti-alpha-synucléine différents, dans lequel un anticorps se lie aux résidus 120 à 140 sur l'alpha-synucléine et l'autre anticorps se lie à pS129 de l'alpha-synucléine.

4. Procédé in vitro selon l'une quelconque des revendications 1-33, dans lequel le fluorophore donneur est sélectionné parmi le Lumi4-Tb (cryptate de Tb2+) ou le cryptate d'europium (cryptate de Eu3+).

5. Procédé in vitro selon l'une quelconque des revendications 1-4, dans lequel le fluorophore accepteur est sélectionné parmi d2, XL665, ou la fluorescéine.

6. Procédé selon l'une quelconque des revendications précédentes, dans lequel le sujet est un humain.
